# EUROPÄISCHE PATENTSCHRIFT

(11) **EP 0 758 342 B1**
(45) Veröffentlichungstag und Bekanntmachung des Hinweises auf die Patenterteilung: **05.01.2000**
(21) Anmeldenummer: 95917963.1
(22) Anmeldetag: 26.04.1995
(51) Int. Cl.: C07K 7/06

(54) **NEUER PEPTIDISCHER WIRKSTOFF UND DESSEN HERSTELLUNG**
NEW PEPTIDE ACTIVE SUBSTANCE AND PRODUCTION THEREOF
NOUVEAU PRINCIPE ACTIF PEPTIDIQUE ET SA PRODUCTION

(30) Priorität: 06.05.1994 DE 4415997
(43) Veröffentlichungstag der Anmeldung: 19.02.1997
(73) Patentinhaber: BASF AKTIENGESELLSCHAFT, 67056 Ludwigshafen (DE)
(72) Erfinder: AMBERG, Wilhelm, 61381 Friedrichsdorf (DE); BERNARD, Harald, 67098 Bad Dürkheim (DE); BUSCHMANN, Ernst, 67069 Ludwigshafen (DE); HAUPT, Andreas, Westboro, MA 01581 (US); JANITSCHKE, Lothar, 67259 Kleinniedesheim (DE); JANSSEN, Bernd, 67063 Ludwigshafen (DE); KARL, Ulrich, 67061 Ludwigshafen (DE); KLING, Andreas, 68239 Mannheim (DE); MÜLLER, Stefan, 67346 Speyer (DE); DE POTZOLLI, Bernd, 67098 Bad Dürkheim (DE); RITTER, Kurt, 69115 Heidelberg (DE); THYES, Marco, 67061 Ludwigshafen (DE); ZIERKE, Thomas, 67459 Böhl-Iggelheim (DE)
(86) Internationale Anmeldenummer: EP9501577
(87) Internationale Veröffentlichungsnummer: WO9530691

(56) Entgegenhaltungen:
- WO-A-93/23424

## Beschreibung

Die Erfindung betrifft einen neuen peptidischen Wirkstoff sowie dessen Herstellung und geeignete Ausgangsstoffe für dessen Herstellung.

In der PCT-Anmeldung WO 93/23424 sind Wirkstoffe auf Peptidbasis beschrieben, die interessante antineoplastische Aktivitäten besitzen. Eine besonders gute Wirkung zeigt das Pentapeptid des Beispiels 234 der genannten Anmeldung, welches folgende Formel besitzt: 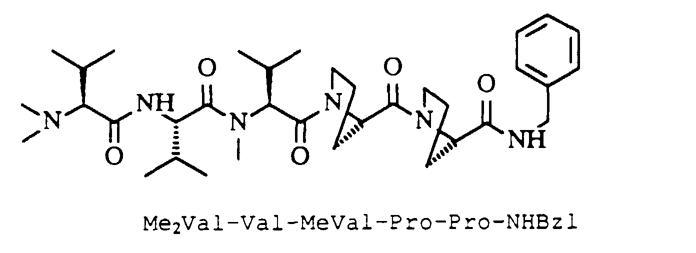 Me₂Val bedeutet N,N-Dimethyl-L-Valin, MeVal steht für N-Methyl-L-Valin. Bzl steht für den Benzylrest.

Das Peptid läßt sich nach der genannten PCT-Anmeldung nach einem Festphasenverfahren ausgehend von Prolin herstellen. Dabei entsteht der Wirkstoff in schlechter Ausbeute und in verunreinigter Form. Eine aufwendige chromatographische Reinigung ist erforderlich. Das Festphase-Verfahren ist zudem nur für die Herstellung kleiner Substanzmengen geeignet. Bisher ist es nicht gelungen, die Substanz des Beispiels 234 der WO 93/23424 in kristalliner Form herzustellen. Der Wirkstoff liegt als Harz vor. Dadurch ist die vollständige Abtrennung von Lösungsmittelresten schwierig. Teure Reinigungsschritte (Sprühtrocknung, Gefriertrocknung) sind erforderlich. Die galenische Weiterverarbeitung der Substanz ist erschwert. Zur Prüfung und Einführung der Substanz sind größere Substanzmengen erforderlich. Zu ihrer Herstellung ist ein technisch realisierbares Verfahren notwendig, das den Wirkstoff möglichst in kristalliner Form liefert.

Es wurde nun ein Verfahren gefunden, das den Wirkstoff racemisierungsfrei in hoher Reinheit ergibt, so daß die Substanz ohne Schwierigkeiten in ein kristallines Salz, das Hydrochlorid, überführt werden kann.

Gegenstand der Erfindung ist ein Verfahren zur Herstellung von Me₂Val-Val-MeVal-Pro-Pro-NHBz1·HCl, welches darin besteht, daß man aus einer Verbindung der Formel

Z-Val-Val-MeVal-Pro-OR¹ II,

worin
- R¹: eine C₁₋₅-Alkylgruppe bedeutet und
- Z: eine Benzyloxycarbonylschutzgruppe darstellt, die am Phenylring substituiert sein kann,

A
   a) am N-Terminus die Schutzgruppe Z abspaltet und an der so erhaltenen Verbindung
      1) die freie Aminogruppe am N-Terminus zweifach methyliert,
      2) die Alkoxygruppe -OR¹ am C-Terminus hydrolysiert und
   b) die so erhaltene Verbindung der Formel

      Me₂Val-Val-MeVal-Pro-OH V

      mit Prolinbenzylamid XII koppelt
      oder
B
   a) am C-Terminus die Alkoxygruppe -OR¹ entfernt und
   b) die so erhaltene Verbindung der Formel

      Z-Val-Val-MeVal-Pro-OH IX

      mit Prolinbenzylamid XII koppelt und die so erhaltene Verbindung der Formel

      Z-Val-Val-MeVal-Pro-Pro-NHBzl XI

      1) am N-Terminus von der Schutzgruppe Z befreit und
      2) an der freien Aminogruppe am N-Terminus zweifach methyliert
und die so erhaltene Verbindung in Ihr Hydrochlorid überführt.

Das Verfahren A verläuft nach folgendem Reaktionsschema:

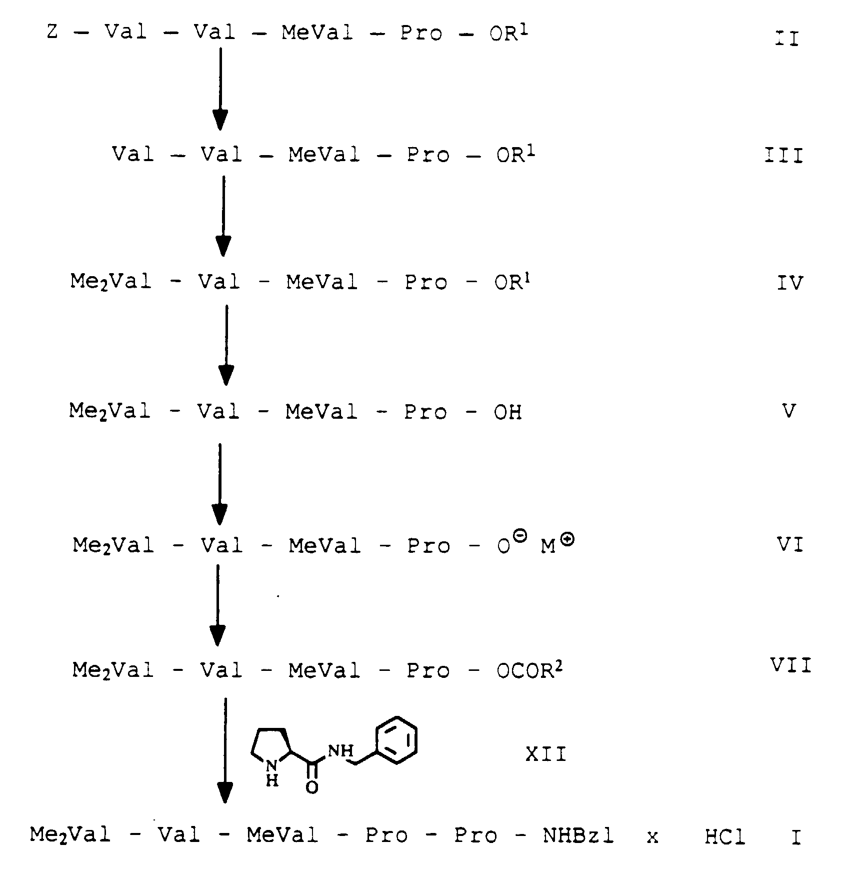

Die Substituenten haben im obigen Formelschema wie überall in der Beschreibung folgende Bedeutungen:
- R¹:: C₁₋₅-Alkyl wie Methyl, Ethyl, n-Propyl, Isopropyl, n-Butyl, tert.-Butyl, Isobutyl, Pentyl und vorzugsweise Methyl und Ethyl,
- R²:: tert.-Butyl, 2-Ethylhexyl, C₁₋₄-Alkoxy wie Methoxy, Ethoxy, Isobutoxy,
- Z:: Benzyloxycarbonyl, am Phenylring gegebenenfalls durch Halogen, C₁-4-Alkyl, C₁₋₄-Alkoxy, C₁₋₄-Acyloxy oder Nitro und insbesondere durch 2-Cl, 3-Cl, 4-Cl, 4-Br, 4-CH₃O-, 4-CH₃COO-, 2-NO₂ und 4-NO₂ substituiert,
M^{⊕} : K^{⊕}, Na^{⊕}, Li^{⊕} oder ein Ammoniumion wie ^{⊕}NH(C₂H₅)₃
- Bzl:: Benzyl
- Me:: Methyl.

Der Tetrapeptidester II wird in einem geeigneten Lösungsmittel, z.B. einem Alkohol - wie Methanol, Ethanol, Isopropanol, Butanol -, einem Ether - wie THF, Dioxan, MTBE - einem Ester - wie Essigsäureethylester -, oder Eisessig gelöst. Nach Zugabe eines geeigneten Katalysators wie z. B. Pd/C oder Pt/C wird bei Temperaturen zwischen 0 und 50°C, bevorzugt zwischen 10 und 30°C, Wasserstoff eingeleitet. Die Wasserstoffeinleitung kann unter Normaldruck oder erhöhtem Druck bis 10bar erfolgen. Die Reaktion kann beschleunigt werden, wenn man einen gewissen Abgasstrom zuläßt. Nach Ende der Wasserstoffaufnahme werden 2 bis 5 Äquivalente Formaldehyd in Form einer wäßrigen Lösung oder auch gasförmig oder in Form von Paraformaldehyd zugegeben. Anschließend wird weiterhin unter den oben beschriebenen Bedingungen Wasserstoff eingeleitet. Danach wird der Katalysator abfiltriert. IV kann gereinigt werden durch Kristallisation als Hydrochlorid aus einem geeigneten Lösungsmittel oder Lösungsmittelgemisch. Bewährt hat sich dabei Isopropanol/Methyl-tert.butylether. Spuren des Z-Tetrapeptidesters II in IV lassen sich auch über extraktive Trennverfahren entfernen.

Die Verseifung des Esters IV erfolgt in einem geeigneten Lösungsmittel wie z. B. einem Alkohol wie Methanol, Ethanol, Isopropanol, einem Ether wie MTBE, THF, Dioxan, einem Kohlenwasserstoff wie Toluol, Xylol, oder einem chlorierten Kohlenwasserstoff wie 1,2-Dichlorethan, Methylenchlorid, Chloroform, mit und ohne Wasserzusatz und mit einer geeigneten Base wie NaOH, KOH, LiOH. Die Esterspaltung kann auch durch Säuren erfolgen. Für R¹ = tert.-Butyl sind dafür besonders CF₃CO₂H und eine Lösung von HCI in Dioxan geeignet.

Die erhaltene Tetrapeptidsäure V muß im folgenden mit Prolinbenzylamid XII zum Pentapeptid I gekoppelt werden. Bei derartigen Kopplungsreaktionen kommt es leicht zur Racemisierung. G. Pettit et al. (J. Am. Chem. Soc. 113, 6692-3 (1991)) verwenden deshalb für eine analoge Kopplung mit der Tetrapeptidsäure V als Kopplungsreagenz DEPC [(OEt)₂POCN]. DEPC ist in größeren Mengen nicht käuflich. Die Methode erfordert deshalb zusätzliche Verfahrensschritte mit giftigen Phosphor- und Cyanidreagenzien.

Cyanidhaltige Abfälle verursachen Entsorgungsprobleme. Das Verfahren ist deshalb für eine technische Durchführung ungeeignet. Eine Peptidkopplungsmethode, die besonders einfach im technischen Maßstab zu realisieren ist, ist die Gemischte-Anhydrid-Methode (s. z.B. J. Meienhofer in The Peptides, Analysis, Synthesis, Biology, Band 1, Academic Press, Orlando, 1979, S. 264-314). Dabei wird die Säure V mit einer geeigneten Base, z.B. einem tertiären Amin wie Triethylamin, N-Methylmorpholin, Dicyclohexylethylamin, Diisopropylethylamin zu VI deprotoniert. Die Ester der Formel IV können auch mit Basen wie NaOH, KOH, LiOH direkt zu den Salzen VI umgesetzt werden. Die Verbindungen VI werden mit einem Säurechlorid CICOR² zum gemischten Anhydrid der Formel VII umgesetzt. Neben Pivaloylchlorid können auch andere Säurechloride wie z.B. 2-Ethylhexansäurechlorid, Chlorameisensäureethylester, Chlorameisensäuremethylester und Chlorameisensäureisobutylester Verwendung finden. Die gemischen Anhydride neigen sehr stark zur Racemisierung (siehe z.B. J. Meienhofer in The Peptides, Band 1, Academic Press, Orlando, 1979, S. 276 ff.).

Die Tetrapeptidsäure V ließ sich nun überraschenderweise nach der Gemischten-Anhydrid-Methode völlig racemisierungsfrei umsetzen. Besonders gute Ergebnisse wurden mit dem gemischten Anhydrid erzielt, das aus V und Pivaloylchlorid hergestellt wurde. Im Gegensatz zu neueren publizierten Ergebnissen (N.L. Benoiton et al., Can. J. Chem. 65, 619-625 (1987)) liefert die Umsetzung mit PIvaloylchlorid, was Selektivität und Ausbeute betrifft, bessere Ergebnisse als die Umsetzung mit Chlorameisensäureestern. Die Herstellung des gemischten Anhydrids VII und die folgende Kopplung mit Prolinbenzylamid führt man bei Temperaturen von -20 bis +5°C in einem geeigneten Lösungsmittel wie Dioxan, NMP, THF, Toluoi, Methylenchlorid, Dimethylformamid durch. Statt des Prolinbenzylamids XII kann auch ein geeignetes Salz dieser Verbindung wie z. B. das Hydrogensulfat, das Methylsulfonat, das Hydrochlorid oder das Hydrobromid Verwendung finden. Dabei muß dann ein weiteres Äquivalent Base, z. B. Triethylamin zugesetzt werden. Nach erfolgter Peptidkopplung und üblicher extraktiver Aufarbeitung wird das Rohprodukt in einem geeigneten Lösungsmittel beispielsweise einem Kohlenwasserstoff - wie Toluol, Xylol -, oder einem Ether - wie Diethylether, THF, Dioxan, Methyl-tertiär-butylether -, einem Keton - wie Aceton, Methylethylketon, Diethylketon, .Cyclohexanon -, oder in chlorierten Lösungsmitteln - wie Methylenchlorid, Chloroform, 1,2-dichlorethan - gelöst. Durch Einleitung von gasförmigem HCl oder Zudosierung einer Lösung von HCl in einem geeigneten Lösungsmittel wie z. B. THF, Methanol, Isopropanol, n-Pentanol, diisopropylether wird das Hydrochlorid I ausgefällt. Besonders bewährt hat sich dabei ein Verfahren, bei dem die freie Base des Pentapeptids zunächst in Methylethylketon gelöst wird und anschließend eine Lösung von HCl in Isopropanol zugegeben wird.

Das Verfahren B läuft nach folgendem Schema ab:

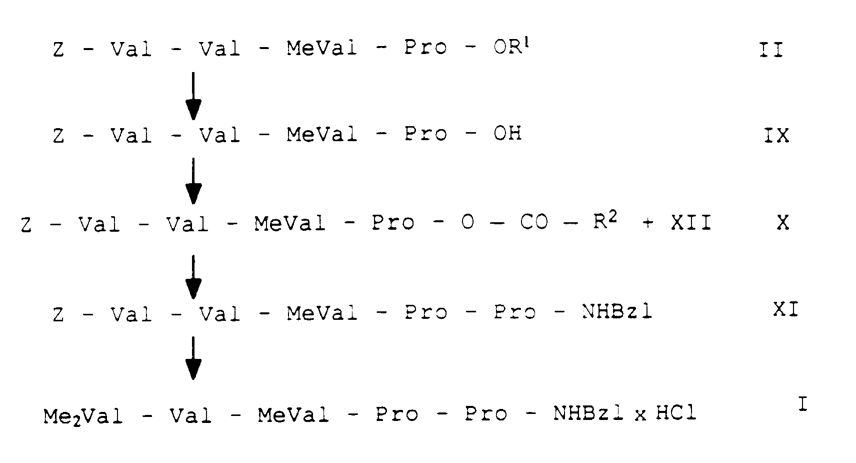

Die Verseifung des Esters II, die Herstellung der gemischten Anhydride X und die Peptidkoppiung zu XI erfolgt in Analogie zur Synthesesequenz IV → V → VI → VII → I. Abspaltung der Z-Schutzgruppe und Dimethylierung zu I erfolgen in Analogie zur Umsetzung II → III → IV.

Auch in der Verfahrensvariante B verläuft die Gemischte-Anhydrid-Methode überraschenderweise racemisierungsfrei. Entgegen den von Benoiton publizierten Ergebnissen werden die besten Ausbeuten mit dem gemischten Anhydrid erzielt, das aus Säure IX und Pivaloylchlorid hergestellt wurde.

Das für die Herstellung des Peptids I benötigte Ausgangsmaterial II läßt sich aus Z-Val-O-CO-R² (XIII) und Val-MeVal-Pro-OR¹ (XIV) herstellen.

Das neue Verfahren liefert Wirkstoff I in kristalliner Form. Das Peptid kann auf einfache Weise durch Umkristallisation weiter gereinigt werden. Aufwendige chromatographische Reinigungsschritte sind nicht erforderlich.

Die Erfindung betrifft auch folgende Vorprodukte zur Herstellung von I:

Z-Val-Val-MeVal-Pro-OR¹ II

Val-Val-MeVal-Pro-OR¹ III

Me₂Val-Val-MeVal-Pro-O-CO-R² VII

Z-Val-Val-MeVal-Pro-OH IX

Z-Val-Val-MeVal-Pro-O-CO-R² X

Z-Val-Val-MeVal-Pro-Pro-NHBzl XI

in denen
R¹ und Z die in Anspruch 3 angegebenen Bedeutungen haben und R² für tert.-Butyl, 2-Ethylhexyl, C₁₋₄-Alkoxy, Methoxy, Ethoxy und Isobutoxy steht.

Die Verbindung I ist wirksam gegen feste Tumoren (Tumoren der Lunge, der Brust, des Darms, der Blase, des Enddarms, der Gebärmutter, der Prostata) gegen Leukämie, Lymphome und andere neoplastische Erkrankungen.

Die folgenden Beispiele erläutern die Erfindung.

### Beispiel 1 (Verfahren A)

### A. Herstellung der Ausgangsmaterialien

### a. Z-Val-Val-MeVal-Pro-OMe (II, R¹ = Me)

In einem 400-l-Hydrierkessel wurden 39,6 kg (83,3 mol) Z-Val-MeVal-Pro-OMe (VIII) in 320 1 Methanol zusammen mit 4 kg 5%iger Palladiumkohle vorgelegt. Anschließend wurae unter Kühlung bei 20 -30°C so lange Wasserstoff eingeleitet, bis in der Reaktionslösung kein Edukt mehr nachzuweisen war. Der Katalysator wurde beim Ablassen des Kesselinhalts abfiltriert. Zur Aufarbeitung wurde in einem 400-l-Emailkessel im Wasserstrahlvakuum bis auf 50 1 aufkonzentriert. Anschließend ließ man 50 1 Toluol zulaufen und extrahierte mit 40 l 2 N HCl. Die Toluolphase wurde noch einmal mit 40 l 1 N Salzsäure nachextrahiert und dann abgelassen. Die gesammelte saure Wasserphase wurde in den Kessel zurückgeführt, mit 40 l Methylenchlorid unterschichtet und anschließend durch Zulauf von 50 %iger Natronlauge unter kräftigem Rühren und Kühlung auf pH 9 gebracht. Nach Phasentrennung wurde die Methylenchloridphase abgelassen und die Wasserphase noch zweimal mit je 40 l Methylenchlorid nachextrahiert. Die gesammelte Methylenchloridlösung des Produkts wird mit Wasser neutral gewaschen. Anschließend wird die Methylenchloridphase auf 90 1 aufkonzentriert. Man erhielt Val-MeVal-Pro-OMe (XIV, R¹=CH₃).
Ausbeute von 24,2 kg = 85,2 %.

In einem 400-l-Kessel werden 17,84 kg (70,88 mol) Z-Valin und 4,59 kg (74,42 mol) Triethylamin in 170 l Methylenchlorid gelöst. Zu dieser Lösung dosiert man bei -5 bis -10°C 8,58 kg (70,88 mol) Pivalinsäurechlorid zu. Nach einer Reaktionszeit von 2 h bei -5°C ließ man bei -5°C eine Lösung von 24,2 kg Val-MeVal-Pro-OMe in 86 l Methylenchlorid zulaufen. Nach weiteren 2 h bei -5°C wurde auf 20°C erwärmt und bei dieser Temperatur 12 h gerührt. Zur Aufarbeitung wurden 50 l Wasser zugegeben. Nach Abtrennung der wäßrigen Phase wurde die organische Phase einmal mit 40 l 2 N Salzsäure und zweimal mit je 40 l 2 N Natronlauge extrahiert. Nach Neutralwaschen der organischen Phase mit Wasser wurde das Lösungsmittel Methylenchlorid abdestilliert und durch 300 l Diisopropylether ersetzt. Die Emulsion des Produktöls wurde zur Kristallisation des Produkts auf 60°C erwärmt, mit Impfkristallen versetzt und 7 h bei 60°C gehalten. Zur Vervollständigung der Kristallisation wurde nacheinander 5 h bei 50°C und 5 h bei 40°C weitergerührt und anschließend auf 20°C abgekühlt. Die Kristallsuspension wurde über einen 120-l-Druckfilter abfiltriert und im Stickstoffstrom getrocknet.
- Ausbeute:: 32,2 kg = 79 %
- Schmelzpunkt:: 134 - 135°C

### b. Prolinbenzylamid-Hydrochlorid (XII x HCl)

Zu einer Lösung von 99,7 g Z-Prolin und 58 ml Triethylamin in 1 l CH₂Cl₂ wurde bei -10 bis -15°C 48,2 g Pivalinsäurechlorid zugetropft. Man rührte 45 min bei -10°C nach und gabanschließend innerhalb 0,5 h bei -10°C 42,8 gBenzylamin in 500 ml CH₂Cl₂ zu. Man rührte 1 h bei Raumtemperaturnach. Die CH₂Cl₂-Lösung wurde anschließend zweimal mit 500 ml Wasser, zweimal mit 500 ml 10 %iger wäßriger NaHCO₃-Lösung, zweimal mit 500 ml Wasser, zweimal mit 500 ml 5 %iger wäßriger Zitronensäure-Lösung und zweimal mit 500 ml Wasser gewaschen, über Na₂SO₄ getrocknet und eingedampft. Es verblieben 120 g Rückstand, der in 200 ml Essigester aufgenommen wurde. Man gab zurEssigesterlösung 1,2 l n-Heptan zu, rührte eine Stunde, saugte abund trocknete bei 50°C im Vakuum.
- Ausbeute:: 110 g, 81,3 %
Fp: 93- 94°C

110 g des so erhaltenen Z-Prolinbenzylamids wurden in 1,5 l Methanol gelöst. Nach Zugabe von 0,5 g Pd/C (10 %ig) wurde Wasserstoff eingeleitet. Die Lösung nahm bei Raumtemperatur innerhalb 1,5 h 0,5 l H₂ auf. Nach Abfiltrieren des Katalysators und Eindampfen verblieben 4,6 g eines gelben Öls.

413 g so erhaltenes Prolinbenzylamid wurden in 400 ml Isopropanol gelost. Man gab 630 ml einer gesättigten Lösung von HCl in Isopropanol zu, rührte die entstandene Suspension 2 h bei 0 - 5°C, saugte ab und wusch zweimal mit 250 ml Isopropanol. Der Rückstand wurde bei 50°C im Vakuum getrocknet. Man erhielt 401 g Prolinbenzylamid-Hydrochlorid; α_{D}²⁰: - 45°

### B. Herstellung des Endprodukts

### a.1

### Me₂Val-Val-MeVal-Pro-OMe x HCI (IV x HCl, R¹= Me)

In einem 400-l-Hydrierkessel wurden 20 kg (34,8 mol) Z-Val-Val-MeVal-Pro-OMe (II, R¹=Me) zusammen mit 2 kg 5 %iger Palladiumkohlé in 200 l Methanol vorgelegt. Anschließend wurde unter Kühlung bei 20°C so lange Wasserstoff eingeleitet, bis in der Reaktionslösung kein Edukt mehr nachzuweisen war. Anschließend fügte man 8,46 kg 37 %ige (104 mol) wäßrige Formaldehyd-Lösung zu und hydrierte bis zur Beendigung der Wasserstoffaufmahme bei 20°C weiter. Der Katalysator wurde beim Ablassen des Kesselinhalts abfiltriert. Zur Aufarbeitung wurde in einem 400-l-Email-Kessel im Wasserstrahl-Vakuum bis auf 50 l aufkonzentriert. Danach wurden 200 l Isopropanol zugegeben und erneut auf 50 l eingeengt. Anschließend löste man in 135 l Methyl-tert.-butylether und fügte unter Kühlung bei 20°C ein Äquivalent isopropanolische HCl zu. Die entstandene Suspension wurde noch 3-4 h bei 20°C und 2 h bei 0-5°C weitergerührt und dann über einen 120-l-Druckfilter filtriert. Der Filterkuchen wurde einmal mit 501 frischem Methyl-tert.-butylether gewaschen.
- Ausbeute:: 16,2 kg = 92,3 %
- Schmelzpunkt:: 224°C (Zersetzung)

### a.2

Die Zwischenstufe Val-Val-MeVal-Pro-OMe (III, R¹=CH₃) war ebenfalls isolierbar, wenn nach der ersten Hydrierstufe wie folgt aufgearbeitet wurde:

Die Reaktionslösung wurde vom Katalysator abgetrennt und eingeengt. Der Rückstand wurde in Essigester aufgenommen. Die Essigesterlösung wurde zweimal mit 2 N Salzsäure extrahiert. Die saure wäßrige Phase wurde mit Natronlauge auf pH 9 gestellt und zweimal mit Methylenchlorid extrahiert. Die Methylenchloridphase wurde anschließend neutral gewaschen und eingedampft.
- HPLC: 96,8 %
¹H-NMR (400 MHz, CDCl₃ / TMSᵢₙₜ):
- δ (ppm):: 0,84 - 1,08 (m, 18H); 1,45 - 1,6 (S, breit, NH₂); 1,85 - 2,15 (m, 4H); 2,18 - 2,38 (m, 3H); 3,15 (s, N-CH₃); 3,25 (d, 1H); 3,65 - 3,75 (m, 1H); 3,73 (s, O-CH₃); 3,9 - 4,05 (m, 1H); 4,38 - 4,45 (m, 1H); 4,73 - 4,83 (m, 1H); 5,12 (d, 1H); 7,9 (d, NH)

### a.3

Die Herstellung von Me2Val-Val-MeVal-Pro-OMe x HCI (IV x HCI, R^{l}=Me) kann auch nach der folgenden Herstellvorschrift erfolgen, bei der auf eine Isolierung und Reinigung der Zwischenstufe Z-Val-Val-MeVal-Pro-OMe (II, R¹=Me) verzichtet wird:

In einem 4-l-Kolben wurden 128 g (0,51 mol) Z-Valin und 55,1 g (0,54 mol) Triethylamin in 1,2 l Methylenchlorid gelöst. Zu dieser Lösung dosierte man bei -5°C bis -10°C 62,1 g (0,51 mol) Pivalinsäurechlorid. Nach einer Reaktionszeit von 2 h bei -5°C ließ man eine Lösung von 174,6 g (0,51 mol) Val-MeVal-Pro-OMe in 0,8 l Methylenchlorid zulaufen, rührte weitere 2 h bei -5°C und dann nach Erwärmung auf 20°C weitere 12 h. Danach wurde der Ansatz mit 370 ml Wasser versetzt. Nach der Phasentrennung wurde die Methylenchloridphase einmal mit 290 ml 2 N Salzsäure, zweimal mit je 290 ml 2 N Natronlauge und dreimal mit 370 ml Wasser gewaschen. Anschließend wurde das Lösungsmittel Methylenchlorid abgedampft und durch 3 l Methanol ersetzt. Zu dieser Lösung gab man eine Aufschlämmung von 30 g 5 %iger Palladiumkohle in 110ml Wasser und hydrierte bei 25°C mittels Begasungsrührer und Wasserstoffbürette bis ein Äquivalent Wasserstoff aufgenommen war. Danach gab man 123 g (1,53 mol) 37 %ige wäßrige Formaldehydlösung zu und hydrierte bis zu Aufnahme weiterer 2 Äquivalente Wasserstoff weiter. Danach wurde vom Katalysator abgetrennt und am Rotationsverdampfer eingedampft. Das verbleibende Öl wurde in 670ml Isopropanol und 2,6 l Methyl-tert.-Butylether gelöst. Zu dieser Lösung gab man ein Äquivalent isopropanolische HCl. Die entstandene Suspension wurde bei 20°C über 12 h weitergerührt und dann abgesaugt. Der Filterkuchen wurde mit wenig Methyl-tert.-butylether gewaschen und anschließend im Vakuumtrockenschrank bei 40°C getrocknet.
- Ausbeute:: 182,8 g = 71 %
- Schmelzpunkt:: 224°C (Zersetzung)

### b. Me₂Val-Val-MeVal-Pro-Pro-NHBzl x HCl (I)

In einem 400-l-Kessel wurden 15,9 kg (31,5 mol) Me₂Val-Val-MeVal-Pro-OMe x HCl (IV x HCl, R¹=CH₃) zusammen mit 140 1 Toluol und 15 1 Methanol vorgelegt. Dazu gab man 3,15 kg (76,38mol) Natriumhydroxid-Pellets. Nach vollständiger Verseifung, d.h. nach 3 h bei 20°C, wurde durch Zugabe von isopropanolischer HCI neutralisiert. Anschließend wurde im Azeotrop mit Toluol bei 100 mbar bis zur Alkohol- und Wasserfreiheit destilliert. Das abdestillierte Lösungsmittel wurde sukzessive durch Toluol ersetzt. Anschließend werden 80 l Methylenchlorid und 6,44 kg (63,0 mol) Triethylamin (Gehalt: 99%) zugegeben, auf -5°C abgekühlt und bei dieser Temperatur 3,84 kg (31,5 mol) Pivalinsäurechlorid zudosiert. Nach zweistündiger Reaktionszeit gab man bei -5°C bis 0°C 7,6 kg (31,5 mol) Pro-NHBz1 x HCI portionsweise zu. Nach 2 h Stehen bei -5°C erwärmte man auf 20°C und ließ noch weitere 6 h reagieren. Anschließend destillierte man bei 500 mbar das zugesetzte Methylenchlorid ab und fügte 80 l Toluol zu. Danach gab man 50 l Wasser zu und stellte den pH-Wert der wäßrigen Phase auf pH 9 ein. Nach kräftigem Rühren wurde die wäßrige Phase abgetrennt und die organische Phase einmal mit 25 l Wasser nachgewaschen. Anschließend wurde die organische Phase zweimal mit je 501 2 N Salzsäure extrahiert. Das Produkt wurde aus der sauren Wasserphase nach Einstellung des pH auf 9 durch 3faches Extrahieren mit je 50 l Methylenchlorid zurückextrahiert. Nach Neutralwaschen der Methylenchloridphase mit Wasser wurde das Methylenchlorid abdestilliert und durch 180 1 Methylethylketon ersetzt. Die Lösung wurde auf 40°C aufgewärmt und mit einem Äquivalent (31,5mol) isopropanolischer HCI versetzt. Die entstandene Suspension wurde auf 60°C aufgewärmt und anschließend ohne weitere Wärmezufuhr 12 h gerührt. Anschließend wurde auf 20°C abgekühlt und weitere 5 h gerührt. Danach wurde auf 5°C abgekühlt und über einen 120-l-Druckfilter filtriert. Der Filterkuchen wurde mit 60 l frischem, 5°C kaltem, Methylethylketon gewaschen. Nach Vortrocknung auf dem Filter wird das Produkt im Vakuumtrockenschrank bei 400C bis zur Gewichtskonstanz getrocknet.
- Ausbeute:: 14,36 kg = 67 %
- Schmelzpunkt:: 214°C (Zersetzung)

### Beispiel 2 (Verfahren B)

### a. Z-Val-VaL-MeVal-Pro-OH (IX)

In einem 2-l-Kolben werden 117 g (0,2 mol) Z-Val-Val-MeVal-Pro-OMe (II, R¹=Me, Beispiel 1Aa) in 900 ml Methanol und 47,5 ml Wasser gelöst. Anschließend wurden 18 g (0,45 mol) Natriumhydroxid-Pellets zugefügt und 12 h bei 20°C gerührt. Zur Aufarbeitung wurden 250ml Wasser zugesetzt und das Methanol abdestilliert. Danach wurde so viel Essigester zugegeben, daß eine klare Phasentrennung erfolgte (ca. 500 ml). Die Essigesterphase wurde abgetrennt. Die wäßrige Phase wurde mit Salzsäure auf pH 1 angesäuert und zweimal mit 500 ml Methylenchlorid extrahiert. Die organische Phase wurde anschließend bis zur Trockne eingedampft.
- Ausbeute:: 105 g = 96,4 %
¹H-NMR (200 MHz, CDCl₃/TMS_{int.})
- δ (ppm):: 0,6 - 1,2 (m, 18H); 1,7 - 2,45 (m, 7H); 3,2 (s, N-CH₃); 3,55 - 3,95 (m, 2H); 4,05 - 4,2 (m, 1H); 4,35 - 4,5 (m, 1H); 4,68 - 4,85 (m, 1H); 4,98 - 5,2 (m, 3H); 5,93 (d, Val-1NH); 7,2 - 7,4 (m, 5H): 7,53 - 7,68 (Val-2NH); 9,6 - 10,2 (s, breit, COOH)

### b. Z-Val-Val-MeVal-Pro-Pro-NHBzl (XI)

5 g Z-Val-Val-MeVal-Pro-OH (8,75 mmol) (IX) wurden in 50 ml CH₂Cl₂ gelöst, 1,79 g (17,5 mmol) Triethylamin zugetropft, auf 10°C abgekühlt und bei dieser Temperatur 1,08 g (8,75 mmol) Pivalinsäurechlorid zugetropft. Nach 2 h Rühren bei 10°C wurde bei dieser Temperatur eine Lösung von 2,11 g (8,75 mmol) Pro-NHBzl x HCI in 10 ml Methanol zugetropft, 2 h bei 10°C und über Nacht bei Raumtemperatur nachgerührt.

Das Reaktionsgemisch wurde 3mal mit je 50 ml Wasser, einmal mit 50ml Wasser bei pH 9 und noch 2mal mit je 50 ml Wasser gewaschen. Die Methylenchlorid-Lösung wurde einrotiert. Als Rückstand verbleiben 5,3 g (81,4 %) weißes kristallines Produkt (Reinheit: 88,7 %).
- Schmelzpunkt:: 118 - 122°C

### c. Me₂Val-Val-MeVal-Pro-Pro-NHBzl x HCI (I)

12 g Z-Val-Val-MeVal-Pro-Pro-NHBzl (XI) wurden in 200 ml Methanol gelöst. Dazu gab man 2 g 5 %ige Palladiumkohle (aufgeschlämmt mit 20 ml Wasser) und hydrierte bei 20°C bis zur Beendigung der Wasserstoffaufnahme. Anschließend gab man 6,5 g 37 %ige wäßrige Formaldehydlösung zu und hydrierte weiter bis zur Beendigung der Wasserstoffaufnahme. Danacn wurde der Katalysator abgetrennt und die Reaktionslösung eingedampft. Der Rückstand wurde in Toluol aufgenommen, wieder eingeengt, nochmals mit 200 ml Toluol versetzt und filtriert. Die toluolische Lösung wurde anschließend 2x mit 50 ml 2 N Salzsäure extrahiert. Die saure wäßrige Phase wurde mit Natronlauge auf pH 9 gebracht und dreimal mit 50 ml Methylenchlorid extrahiert. Die Methylenchloridphase wurde mit Wasser neutral gewaschen und eingeengt. Die Rohbase wurde in einer Lösung von 150 ml Methylethylketon und 7,5 ml Isopropanol aufgenommen. Aus dieser Lösung fällte man durch Zugabe von 4 g 25%iger isopropanolischer HCI bei 40°C das Produkt-Salz aus. Die Suspension wird 3 h bei 20°C und eine Stunde bei 0-5°C nachgerührt und anschließend abgesaugt.
- Ausbeute:: 7,1 g
- Gehalt:: 99,1 % (HPLC-Flächenprozent)
- Schmelzpunkt :: 214°C (Zersetzung)

## Patentansprüche

1. Me₂Val-Val-MeVal-Pro-Pro-NHBzl·HCl (I).

2. Me₂Val-Val-MeVal-Pro-Pro-NHBzI·HCI (I) in kristalline Form.

3. Verfahren zur Herstellung von Me₂Val-Val-MeVal-Pro-Pro-NHBzl·HCI, dadurch gekennzeichnet, daß man aus einer Verbindung der Formel II
Z-Val-Val-MeVal-Pro-OR¹ II
worin
R¹ eine C₁₋₅-Alkylgruppe bedeutet und
Z eine Benzyloxycarbonylschutzgruppe darstellt, die am Phenylring substituiert sein kann,
A
a) am N-Terminus die Schutzgruppe Z abspaltet und an der so erhaltenen Verbindung
1) die freie Aminogruppe am N-Terminus zweifach methyliert,
2) die Alkoxygruppe -OR¹ am C-Terminus hydrolysiert und
b) die so erhaltene Verbindung der Formel
Me₂Val-Val-MeVal-Pro-OH V
mit Prolinbenzylamid kuppelt
oder
B
a) am C-Terminus die Alkoxygruppe -OR¹ entfernt und
b) die so erhaltene Verbindung der Formel
Z-Val-Val-MeVal-Pro-OH IX
mit Prolinbenzylamid kuppelt und die so erhaltene Verbindung der Formel
Z-Val-Val-MeVal-Pro-Pro-NHBzl XI
1) am N-Terminus von der Schutzgruppe Z befreit und
2) an der freien Aminogruppe am N-Terminus zweifach methyliert
und die so erhaltene Verbindung in ihr Hydrochlorid überführt.

4. Verfahren gemäß Anspruch 3, dadurch gekennzeichnet, daß man die Tetrapeptidcarbonsäuren der Formeln V bzw. IX zwecks Kupplung mit Prolinbenzylamid zunächst in gemischte Anhydride mit Pivalinsäure überführt.

5. Vorprodukte zur Herstellung der Verbindung I gemäß Anspruch 1 der Formeln
Z-Val-Val-MeVal-Pro-OR¹ II
Val-Val-MeVal-Pro-OR¹ III
Me₂Val-Val-MeVal-Pro-O-CO-R² VII
Z-Val-Val-MeVal-Pro-OH IX
Z-Val-Val-MeVal-Pro-O-CO-R² X
Z-Val-Val-MeVal-Pro-Pro-NHBzl XI
in denen
R¹ und Z die in Anspruch 3 angegebenen Bedeutungen haben und
R² für tert.-Butyl, 2-Ethylhexyl, C₁₋₄-Alkoxy, Methoxy, Ethoxy oder Isobutoxy steht.

## Revendications

1. Me₂Val-Val-MeVal-Pro-Pro-NHBzl·HCl (I).

2. Me₂Val-Val-MeVal-Pro-Pro-NHBzl·HCl (I) à l'état cristallisé.

3. Procédé pour la préparation de Me₂Val-Val-MeVal-Pro-Pro-NHBzl.HCl, caractérisé par le fait que, partant d'un composé de formule II
Z-Val-Val-MeVal-Pro-OR¹ II
dans laquelle
R¹ représente un groupe alkyle en C1-C5 et
Z un groupe protecteur benzyloxycarbonyle qui peut être substitué sur le cycle phényle,
A
a) on élimine le groupe protecteur Z de l'azote terminal, et sur le composé ainsi obtenu,
1) on méthyle deux fois le groupe amino libre de l'azote terminal,
2) on hydrolyse le groupe alcoxy -OR¹ du carbone terminal et
b) on condense le composé ainsi obtenu, de formule
Me₂Val-Val-MeVal-Pro-OH V
avec le benzylamide de la proline,
ou bien
B
a) on élimine le groupe alcoxy -OR¹ du carbone terminal et
b) on condense le composé ainsi obtenu, de formule
Z-Val-Val-MeVal-Pro-OH IX
avec le benzylamide de la proline, ce qui donne le composé de formule
Z-Val-Val-MeVal-Pro-Pro-NHBzl XI
1) dont on élimine le groupe protecteur Z de l'azote terminal et
2) dont on méthyle deux fois le groupe amino libre de l'azote terminal,
et on convertit le composé ainsi obtenu en son chlorhydrate.

4. Procédé selon la revendication 3, caractérisé par le fait que, pour la condensation avec le benzylamide de la proline, on convertit d'abord les acides tétrapeptide-carboxyliques de formules respectives V et IX en anhydrides mixtes avec l'acide pivalique.

5. Produits intermédiaires de la préparation des composés I selon la revendication 1, de formules
Z-Val-Val-MeVal-Pro-OR¹ II
Val-Val-MeVal-Pro-OR¹ III
Me₂Val-Val-MeVal-Pro-O-CO-R² VII
Z-Val-Val-MeVal-Pro-OH IX
Z-Val-Val-MeVal-Pro-O-CO-R² X
Z-Val-Val-MeVal-Pro-Pro-NHBzl XI
dans lesquelles
R¹ et Z ont les significations indiquées dans la revendication 3 et
R² représente un groupe tert-butyle, 2-éthylhexyle, alcoxy en C1-C4, méthoxy, éthoxy ou isobutoxy.

## Claims

1. Me₂Val-Val-MeVal-Pro-Pro-NHBzl·HCl (I).

2. Me₂Val-Val-MeVal-Pro-Pro-NHBzI·HCl (I) in crystalline form.

3. A process for preparing Me2Val-Val-MeVal-Pro-Pro-NHBzl·HCl, which comprises in a compound of the formula II
Z-Val-Val-MeVal-Pro-OR¹ II
where
R¹ is C₁₋₅-alkyl and
Z is a benzyloxycarbonyl protective group which may be substituted on the phenyl ring,
A
a) eliminating the protective group Z on the N terminus, and in the resulting compound
1) dimethylating the free amino group on the N terminus,
2)hydrolyzing the alkoxy group -OR¹ on the C terminus and
b)coupling the resulting compound of the formula
Me₂Val-Val-MeVal-Pro-OH V
with proline benzylamide
or
B
a)removing the alkoxy group -OR¹ on the C terminus and
b)coupling the resulting compound of the formula
Z-Val-Val-MeVal-Pro-OH IX
with proline benzylamide, and in the resulting compound of the formula
Z-Val-Val-MeVal-Pro-Pro-NHBzl XI
1) removing the protective group Z on the N terminus and
2)dimethylating the free amino group on the N terminus
and converting the resulting compound into its hydrochloride.

4. A process as claimed in claim 3, wherein the tetrapeptidecarboxylic acids of the formulae V and IX are initially, for the purpose of coupling with proline benzylamide, converted into mixed anhydrides with pivalic acid.

5. A precursor for preparing the compound I as claimed in claim 1, of the formula
Z-Val-Val-MeVal-Pro-OR¹ II
Val-Val-MeVal-Pro-OR¹ III
Me₂Val-Val-MeVal-Pro-O-CO-R² VII
Z-Val-Val-MeVal-Pro-OH IX
Z-Val-Val-MeVal-Pro-O-CO-R² X
Z-Val-Val-MeVal-Pro-Pro-NHBzl XI
where
R¹ and Z have the meanings stated in claim 3, and
R² is tert-butyl, 2-ethylhexyl, C₁₋₄-alkoxy, methoxy, ethoxy or isobutoxy.
